# EUROPEAN PATENT APPLICATION

(11) **EP 2 077 132 A1**
(43) Date of publication of application: **08.07.2009**
(21) Application number: 08000010.2
(22) Date of filing: 02.01.2008
(51) Int. Cl.: A61M 15/00

(54) **Dispensing device, storage device and method for dispensing a formulation**

(71) Applicant: Boehringer Ingelheim Pharma GmbH & Co. KG, 55216 Ingelheim am Rhein (DE)
(72) Inventor: Dunne, Stephen T., Suffolk, IP14 3AE (GB); Moser, Achim, 55216 Ingelheim am Rhein (DE)
(74) Representative: Gesthuysen, von Rohr & Eggert

(57) **Abstract**

A dispensing device, a storage device and a method for dispensing a medical formulation are proposed. Multiple doses of the formulation are stored in a carrier having multiple storage members, each storage member containing a single dose. Each storage member comprises one duct or nozzle for dispensing the respective dose. The dispensing direction of the ducts or nozzles is directed in a lengthwise or indexing direction of the carrier. The storage device of the carrier is a peelable blister strip, wherein the ducts or nozzles can be opened one after the other by peeling the blister strip.

## Description

The present invention relates to a dispensing device for dispensing a preferably medical formulation, in particular containing or consisting of a drug or mixture of drugs, according to the preamble of claim 1, to a storage device for a preferably medical formulation, in particular containing or consisting of a drug or mixture of drugs, according to the preamble of claim 15, and to a method for dispensing a preferably medical formulation, in particular containing or consisting of a drug or mixture of drugs, according to the preamble of claim 25.

Drugs delivered through dispensing devices, in particular inhalers, are intended to optimally target specific sites in the pulmonary system. These sites include the nasal passages, the throat, and various locations within the lungs, such as the bronchi, bronchioles and alveolar regions. The ability to deliver drugs to a target area depends inter alia on the aerodynamic sizes of the particles or droplets. As currently believed to be understood, particles having an aerodynamic diameter of less than 2 µm are considered to be potentially optimal for deposition in the alveolar region of the lung. Particles that have an aerodynamic diameter of between 2 and approximately 5 µm may be more suitable for delivery to the bronchiole or bronchi regions, Particles with an aerodynamic size range greater than 6 µm, and more preferably 10 µm, are typically suitable for delivery to the laryngeal region, throat or nasal passages.

In most cases, it is desired to achieve a high inhalable fraction and a high delivery efficiency, i.e. the fraction of the initial dose of drug that reaches the desired region, in particular in the lung. This depends on various factors, in particular on the characteristics of the generated spray plume, such as propagation velocity of the plume, particle size and its distribution, fraction of small particles, fraction of gas or the like. In the present invention, the desired spray plume characteristics include preferably a small particle size, a high fraction of drug particles with a diameter of 6 µm or less, a low propagation velocity and/or a long duration of spray generation and possible inhalation.

The present invention relates to the dispensing of a preferably medical formulation. The term "formulation" relates in particular to powder, but may include or relate to liquid as well. Consequently, the fine "particles" may be either solid or liquid. The term "liquid" has to be understood preferably in a broad sense covering inter alia solutions, suspensions, suslutions, mixtures thereof or the like. More particularly, the present invention relates to the dispensing of formulations for inhalation, such as medical formulations containing or consisting of at least one drug.

In the following, the description will focus mainly on powder formulations. However, the same applies for liquid formulations.

In particular, the present invention is concerned with dry powder inhalers for the delivery of drugs to the lungs. Many dry powder inhalers are on the market or have been proposed. There are two main types, namely the passive ones and the active ones. In passive inhalers all the energy required for de-agglomerating the powder and transferring the powder to the lungs is provided by the breathing of a user, respectively the patient. In active inhalers there is an additional source of energy to help to de-agglomerate the powder.

Most powder inhalers are of the passive type where the powder is inhaled by the patient without the aid of an additional energy source. The problem with passive inhalers is that the inhalable fraction, or the proportion of powder that actually enters the lungs, is largely dependent on the breathing of the patient. The de-agglomeration of the powder and hence the inhalable fraction is a function of the flow rate of inhaled air through the device and, therefore, varies greatly from patient to patient.

Dry powder inhalers are subdivided into single dose and multi-dose devices or inhalers. Multi-dose inhalers are further subdivided into pre-metered types where the doses are stored individually and into metering inhalers where each powder dose is metered in the device.

Multi dose pre-metered inhalers have the advantage that the single doses are metered under strict factory conditions and the powder can quite easily be isolated from the atmosphere. In many applications the active drug powder is mixed with a carrier such as lactose. Both the active drug and lactose tend to absorb humidity from the atmosphere which makes it stick together and difficult to de-agglomerate.

The present invention relates in particular to an active, gas (preferably air or HFA gases such as 134a) powered, pre-metered multi-dose dispensing device for dispensing a formulation containing or consisting of a drug, such as a dry powder inhaler.

US 4,627,432 A discloses a device for administering medicaments to patients, namely an inhaler. The inhaler comprises a disk-like blister pack having a plurality of blister pockets arranged in a circle. Each blister pocket contains a dose of the powder. A plunger can open a blister pocket. When a blister is opened, the medicament can be withdrawn by a patient inhaling through a mouthpiece.

It is difficult to empty the respective blister pocket completely during a dispensing operation. Incomplete emptying results in decreased delivery efficiency. Some powder may be lost in the inhaler and not dispensed because the known solutions require relatively long paths for the powder until the powder reaches a nozzle and is actually dispensed. This might reduce the delivery efficiency further. In addition, de-agglomeration of the powder is difficult and the powder may block an outlet nozzle that is used for dispensing multiple doses one after the other.

WO 2007/062721 A1, which forms the starting point of the present invention, discloses a dispensing device for dispensing a preferably medical formulation as a spray. The dispensing device is adapted to receive or comprises a storage device. The storage device comprises a carrier with multiple cavities. Each cavity comprises a moveable insert. Each insert comprises a storage chamber with a dose of the formulation, and a duct for dispensing the respective dose. The cavities are sealed. The sealings can be opened by pushing the respective insert through the associated sealing. Then, the respective dose is dispensed by pressurized air supplied to the insert.

Object of the present invention is to provide an improved dispensing device, storage device and method for dispensing a preferably medical formulation, in particular wherein a very simple design of the storage device and/or simple opening of the storage device can be achieved.

The above object is achieved by a dispensing device according to claim 1, by a storage device according to claim 15 or by a method according to claim 25. Preferred embodiments are subject of the subclaims.

According to a first aspect of the present invention, the dispensing direction of the ducts or nozzles of storage members located on or formed by a carrier is directed at least essentially in a lengthwise or indexing direction of the storage device or carrier. This allows in particular a very simple construction and/or opening.

According to a second aspect of the present invention, the storage device or carrier, in particular a blister strip or the like, is a peelable blister strip, wherein the ducts or nozzles can be opened one after the other by peeling or cracking or tearing the blister strip. This allows a very simple construction and/or opening.

According to a third aspect of the present invention, the storage members are separate parts that are fixed on the carrier and cannot be moved relatively to the carrier. The storage members or its ducts or nozzles are closed by a preferably common sealing or cover layer. The storage members, ducts or nozzles can be opened one after the other by opening or peeling the sealing or cover layer. This allows a very simple construction and/or opening.

Alternatively or additionally, the storage members or its ducts or nozzles can be opened by pushing or cutting or bending or folding the carrier or blister strip. Preferably, the storage members are rigid, and the carrier / blister strip is flexible. However, the storage members could also be formed only by the flexible carrier.

It has to be noted that each storage member preferably comprises an individual duct or nozzle for dispensing the respective dose. In particular, a spray is directly or only formed by or directly after the duct or nozzle. The duct or nozzle may dispense the generated spray in to a mouthpiece for inhalation by a user or patient. However, any further spray forming means is preferably not provided.

Further aspects, advantages and features of the present invention will be apparent from the claims and following detailed description of preferred embodiments. In the drawings show:
- Fig. 1: a schematic sectional view of a dispensing device with a storage device according to the present invention during dispensing;
- Fig. 2: a top view of a part of the storage device with multiple storage members;
- Fig. 3: a schematic sectional view of a part of the storage device with one storage member;
- Fig. 4: a schematic sectional view of a part of the storage device according to an other embodiment;
- Fig. 5: a schematic sectional view of a part of the storage device according to a further embodiment;
- Fig. 6: a schematic sectional view of a part of the storage device according to still an other embodiment;
- Fig. 7: a schematic sectional view of a part of the storage device shown in Fig. 1 to 3 when pierced to discharge a formulation; and
- Fig. 8: a sectional view perpendicular to Fig. 7.

In the Fig., the same reference signs are used for same or similar components, wherein same or similar characteristics, features or advantages are or can be realized or achieved even if a repeated discussion is omitted. Further, the features and aspects of the different embodiments can be combined in any desired manner and/or used for other dispensing devices or methods for dispensing in particular medical formulations for inhalation.

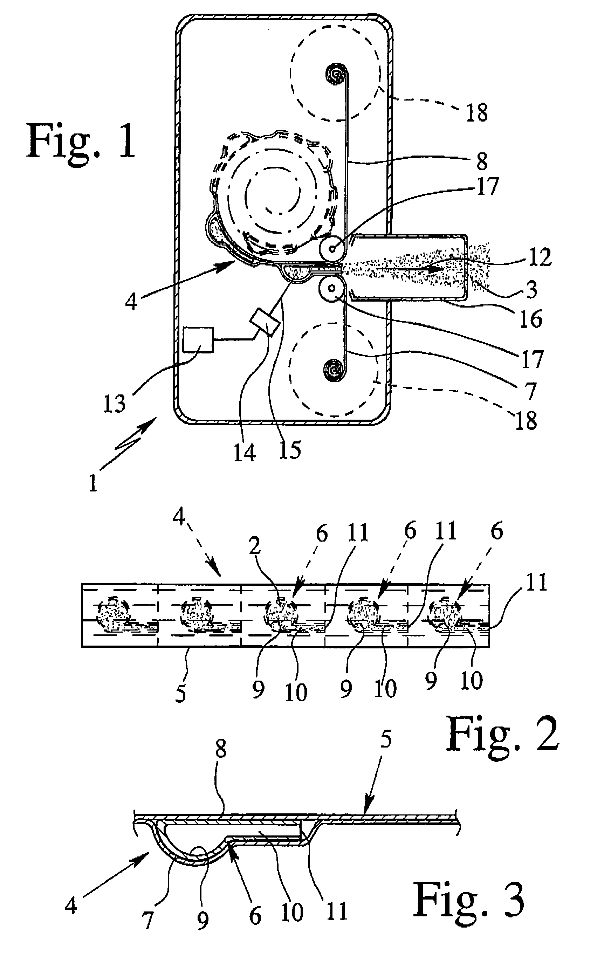

Fig. 1 shows in a schematic sectional view - for illustration purposes not in scale - a dispensing device 1 according to the present invention. The dispensing device 1 is an active device, in particular gas powered. Here, the dispensing device 1 is a preferably oral or nasal inhaler, in particular a dry powder inhaler, for a user, respectively the patient (not shown).

The dispensing device 1 may be used for dispensing any formulation 2 as defined in the introductory part of the description. In particular, a medical formulation 2 or a formulation 2 for inhalation will be used. The formulation 2 preferably contains or consists of at least one drug. When the formulation 2 is dispensed, a spray 3 is generated as indicated in Fig. 1. The spray 3 includes fine particles (solid and/or liquid) of the formulation 2 and preferably has the desired spray plume characteristics.

The formulation 2 may be a powder or a liquid, in particular a solution, a suspension or any mixture thereof, i.e. a so-called suslution. Preferably, when different drugs are dispensed simultaneously, a suslution may be used. The principle of the suslution is based on that different drugs may be combined in one formulation simultaneously as a solution and as a suspension. In this respect, reference is made to EP 1 087 750 A1 which is incorporated herein as additional disclosure in this respect.

Preferably, the formulation 2 is a powder. The powder may be a pure drug or a mixture of at least two drugs. In addition, the powder may contain at least one other material, in particular a drug carrier such as lactose. In the following, the description focuses on powder as formulation 2. However, the description applies in a similar manner if a liquid formulation 2 is used.

Preferably the mean diameter of the powder particles is about 2 to 7 µm, in particular 6 µm or less. This applies in particular if the powder does not contain any drug carrier such as lactose.

If the powder contains a drug carrier, such as lactose, and at least one drug, the powder 2 may have a particle size of 20 to 300 µm, in particular about 30 to 60 µm. However, the de-agglomeration, which will be described later in more detail, may result even in this case in a spray 3 with a smaller particle size, e.g. of about 10 to 50 µm, preferably of about 10 µm or less. In particular, the drug may be separated from the drug carrier during de-agglomeration so that primarily the drug will be inhaled due to its small particle size of about 2 to 6 µm and the larger drug carrier will be swallowed when using the dispensing device as an inhaler. Alternatively or additionally, breaking or opening of the drug carrier is possible during de-agglomeration.

The diameters mentioned above and below may be understood as mass medium aerodynamic diameters and/or may apply to the particle size or a fraction of the particles of the spray 3.

The dispensing device 1 is adapted to receive or comprises a storage device 4 for storing preferably multiple and pre-metered doses of the formulation 2. The storage device 4 may be integrated into the dispensing device 1 or form part of the dispensing device 1. Alternatively, the storage device 4 may be a separate part that can be inserted or connected with the dispensing device 1 and optionally replaced.

The storage device 4 comprises a carrier 5 with storage members 6, as shown also in the top view of Fig. 2 of a part of the storage device 4. In particular, the carrier 5 may comprise 20 to 120, preferably 30 to 60 storage members 6, Each storage member 6 contains preferably one pre-metered dose of the formulation 2. However, each storage member 6 may also contain more than one formulation 2, i.e. different formulations 2. Additionally or alternatively, different storage members 6 may contain different formulations 2. In the present invention, "different" means in particular that the formulations 2 differ in at least one of the composition, the drug, the dose or amount, the concentration, and consistence of the formulation 2, e.g. liquid or dry or powder.

The storage device 4 or carrier 5 is preferably a blister strip.

In the present embodiment, the carrier 5 comprises or consists of a base layer 7 and a sealing or cover layer 8, as shown in Fig. 3 representing a schematic sectional, longitudinal view of a part of the storage device 4 with one storage member 6.

In the present embodiment, the storage members 6 are preferably formed or made as separate parts. In particular, the storage members 6 are mounted or fixed on the carrier 5 or inserted or held between the two layers 7 and 8 before and/or during dispensing.

Before use or opening, the storage members 6 are sealed by the base layer 7 and/or cover layer 8. In the present embodiment, the base layer 7 is indented and/or follows the contour of the preferably bulky storage members 6. The storage members 6 preferably end at least essentially flat in the main plane of the base layer 7. The cover layer 8 is preferably flat and forms a sealing or cover so that the storage members 6 are sealed from each other and/or hermetically against the atmosphere.

The base layer 7 may be formed by any suitable material, in particular plastic or the like, most preferably by any suitable composite material.

The cover layer 8 may be formed by any suitable material, in particular aluminum foil, a plastic/AC composite, a composite foil or any other material suitable for humidity protection and/or gas tight sealing.

However, other constructions are possible. In particular, the base layer 7 may be flat and the cover layer 8 may be indented/embossed, or vice versa. Alternatively, both layers 7 and 8 may be indented and/or non-flat.

Alternatively or additionally, the layers 7 and 8 and/or other parts of the carrier 5 may form the storage members 6.

The storage device 4, carrier 5 or blister strip is preferably longitudinal and/or band-like.

In particular, the storage members 6 are located one after the other in a row and spaced in lengthwise or longitudinal direction of the storage device 4, carrier 5 or blister strip.

Preferably, each storage member 6 is made of foil, plastics, ceramics and/or composite material, in particular of thermoplastics or thermoplastic elastomers and for sealings of elastomers or silicone.

Each storage member 6 may form a preferably block-like unit and/or may be rigid. Alternatively, the storage members 6 may be flexible. In particular, each storage member 6 may be a unitary unit or consists of multiple elements. Each storage member 6 may be a molded element, a cartridge, a blister, a capsule, a container or the like.

In the following, a preferred construction of one storage member 6 is explained. Preferably, all storage members 6 are identical. However, it is also possible that all or some storage members 6 are different. For example, two or more groups of different storage members 6 can be provided. It is possible that one group has a different dose or different formulation 2 than the other group. For example, the storage members 6 of the different groups could be arranged alternately one after the other so that a patient or user may use for example each morning a storage member 6 of one group and each evening a storage member 6 of the other group.

Each storage member 6 comprises a storage chamber 9 for the formulation 2.

Each storage member 6 further comprises a duct 10 or the like for discharging the formulation 2 during the dispensing operation. The formulation 2 is dispensed through the duct 10 during the dispensing operation, in particular for de-agglomerating the powder and/or forming the spray 3.

The duct 10 can comprise or form a nozzle 11 preferably at the outlet. Alternatively, the nozzle 11 or any other suitable nozzle arrangement could be used instead of or in any other combination with duct 10.

Preferably, the duct 10 / nozzle 11 is formed integrally with the storage chamber 9 and/or by a base member of the storage member 6, in particular by a recess, grove or the like in the base member 11, and by an associated cover member of the storage member 6. In particular, the duct 10 forms a channel from the storage chamber 9 to an outlet of the storage member 6 for discharging the formulation 2 as spray 3 as shown in Fig. 1.

Preferably, the duct 10 / nozzle 11 dispenses the dose of formulation 2 of the respective storage member 6 in a dispensing direction as indicated by arrow 12 in Fig. 1.

Preferably, the dispensing direction 12, the duct 10 and/or the nozzles 11 are directed in a lengthwise or indexing direction of the storage device 4 / carrier 5 / blister strip. This allows a very simple and compact design of the dispensing device 1 and/or storage device 4.

Preferably, the storage members 6 / ducts 10 / nozzles 11 are opened one after the other - i.e. individually - by peeling or cracking or tearing the blister strip, in particular the cover layer 8 and/or base layer 7 or any part thereof, so that the respective doses of the formulation 2 can be dispensed one after the other.

Preferably, the formulation 2 is dispensed by gas pressure, in particular air pressure, generating a gas/air stream (active inhaler). However, it is also possible to dispense the formulations 2 by means of an air stream generated by breathing in of a user or patient (not passive inhaler).

In the present embodiment, the dispensing device 1 is active and uses pressurized gas to force the formulation 2 through the duct 10 / nozzle 11 to de-agglomerate the powder and/or to generate the spray 3. Preferably, the dispensing device 1 comprises a means for providing pressurized gas, in the present embodiment an air pump 13 which can preferably be actuated or operated manually. In particular, the air pump 13 comprises or is formed by a bellows. But, it could be also a piston-cylinder-arrangement. Instead of the air pump 13, the means for providing pressurized gas can be e.g. a capsule, container or the like containing pressurized or liquefied gas for powering the dispensing device 1, i.e. dispensing the formulation 2 as desired.

The air pump 13 may provide a gas pressure of less than 500 or 400 kPa, in particular about 50 to 200 kPa. This is preferably sufficient for operating the dispensing device 1. If liquefied gas or a container with pressurized gas is used, the gas pressures might range from 100 kPa to about 700 kPa. Then, the pressure may be reduced or throttled to the preferred pressure range before supplying the gas to the storage device 4, in particular the storage chamber 9 of the respective storage member 6.

Preferably, all pressure values mentioned in the present description are gauge pressures, i.e. pressure differences. All pressure values relate to the pressure in a gas storage such as a container with pressurized or liquefied gas or provided by air pump 13 or relate to the pressures acting in the storage chamber 9 and/or in the duct 10.

Fig. 1 shows schematically that the dispensing device 1 comprises a mechanism 14 for connecting the storage members 6 individually to the gas supply, in particular to the air pump 13. The mechanism 14 comprises preferably a piercing element 15, such as a hollow needle, or any other suitable connecting element.

The connecting mechanism 14 is preferably designed such that it open 5 or pierces the storage chamber 9 of the storage members 6 that shall be emptied next. In particular, the piercing element 15 can penetrate the base layer 7 and/or cover layer 8 and the wall of the storage chamber 9 so that gas can be supplied into the storage chamber 9 to generate a gas stream which entrains the formulation 2 in the storage chamber 9 and dispenses the formulation 2 through the duct 10 / nozzle 11 and generates the spray 3 as schematically shown in Fig. 1.

Actuation and/or movement of the connecting mechanism 14 and/or piercing element 15 can be combined with the actuation of the means for pressurizing gas (air pump 13) or operated separately.

Preferably the storage chamber 9 forms a mixing chamber for mixing the gas with the powder. The chamber 9 is preferably designed such that the gas can generate swirls or eddies for better mixing the powder with the gas. Preferably, the chamber 9 is substantially circular in cross section, in particular cylindrical. However, other shapes are also possible.

Further, the chamber 9 is formed with no sharp edges, corners or the like, but has a smooth contour so that the gas can sweep all chamber surfaces to prevent powder accumulating on said surfaces and to ensure or allow complete discharge of the powder. In particular, the gas inlet formed by the piercing element 15 or any other supply element is located opposite and/or offset to the outlet, i.e. duct 10 and/or nozzle 11, with regard to the axial or outlet direction.

The duct 10 is preferably at least essentially tangentially connected to the storage chamber 9 in the embodiment. Preferably, the duct 10 is connected at one axial end of the preferably cylindrical chamber 9, and the gas inlet is inserted to the other axial end of the chamber 9. In particular, the gas inlet is connected tangentially to the storage chamber 10, such that swirls are generated in the chamber 9 with a swirl direction supporting discharge of the mixture of gas and formulation 2 through the duct 10 which connects preferably at least essentially tangentially to the rotational direction of the swirl.

During the dispensing operation, the spray 3 is preferably directly generated by the respective storage member 6 or its duct 10 / nozzle 11 and outputted into a mouthpiece 16 of the dispensing device 1 as shown in Fig. 1 for inhalation by a patient or user (not shown).

The powder will be discharged - in particular forced through the duct 10 - preferably with a comparatively low gas pressure (preferably less than 300 kPa, in particular about 50 to 200 kPa). This low gas pressure which is preferably significantly lower than the gas pressures usually used in the prior dispensing devices enables a respectively low discharge velocity and, therefore, a low spray 3 with slow propagation velocity.

After dispensing one dose or for dispensing the next dose, the piercing element 15 will be withdrawn from the connected storage member 6. During this opposite movement or by a separate actuation or during the movement for connecting the next storage member 6 to the gas supply, the carrier 5 will be indexed one step further to the next storage member 6.

In the shown embodiment, the carrier 5, base layer 7 and/or cover layer 8 may be used to feed or index or move the storage device 4 from one storage member 6 to the next storage member 6.

In particular, the base layer 7 and/or cover layer 8 can be peeled or pulled, preferably in at least essentially opposite directions or in directions inclined to each other. The storage device 4 / carrier 5 and/or layers 7 or 8 may be guided by guide rollers 17 or any other suitable guiding elements and/or wound up/or folded by suitable mechanisms, in particular spooling devices 18 or the like.

Preferably, the pooling of the carrier 5 and/or of the base layer 7 and/or cover layer 8 is preferably the only drive to index the storage device 4 one step further or to the next storage member 6. However, other constructional solutions are possible as well.

The unused storage device 4 may be wound up in the dispensing device 1, e.g. together with the carrier 5, in particular base layer 7.

According to another (not shown) embodiment, the duct 10 can also be used as a reservoir (storage chamber 9) for the formulation 2. In this case, the separate storage chamber 9 is not required. Then, the duct 10 is designed to enable sufficient mixing of the gas with the formulation 2 and sufficient de-agglomeration of the powder formulation 2.

Preferably, the spray 3 has a mean velocity (taken 20 cm from the outlet 15 or mouthpiece 16) of less than 2 m/s, in particular less than 1 m/s. Preferably, the mean duration of the spray 3 is at least 0.2 or 0.3 s, in particular about 0.5 to 2 s.

In general, a secondary packaging may be used for packing and protecting the storage device 4 / carrier 5, in particular for storage purposes before storage membering the storage device 4 / carrier 5 into the dispensing device 1. Additionally the whole device 1 including storage device 4 / carrier 5 may be stored in a secondary water vapour proof packaging.

In general, the dispensing devise 1 may be breath activated, in particular wherein the formulation 2 is only released after the patient's or user's inhalation rate has reached a predetermined level, preferably by the use of a pressure sensitive means, such as a bursting element, membrane or valve, or any other mechanism.

In general, the storage member 6 will be preformed and/or formed separately before connected with or integrated into the carrier 5. Preferably, the storage members 6 are separately or individually sealed, in particular after filling and before integration into or connection with the carrier 5.

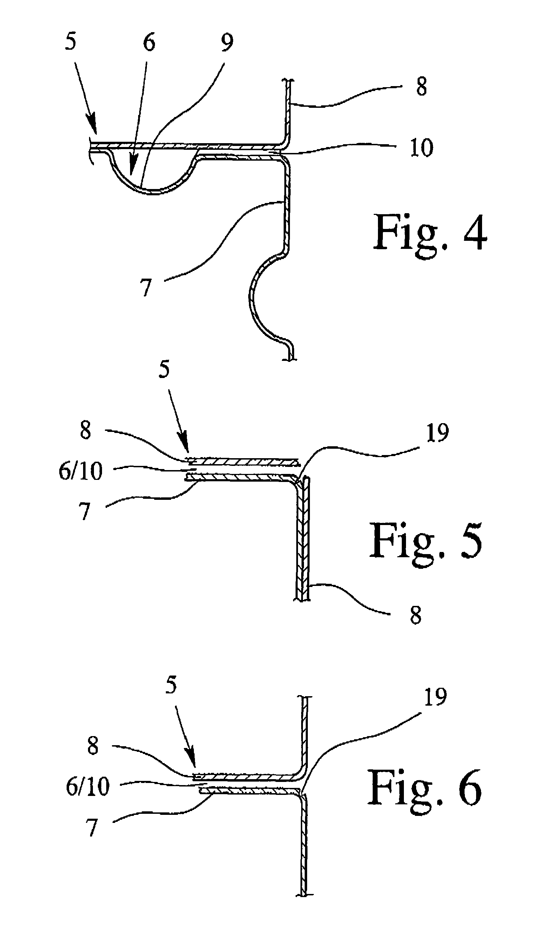

However, the storage member 6 can also be formed directly by the carrier 5. This is realized in the other embodiment shown in a schematic partial section of Fig. 4. Here, the storage members 6 are preferably formed by indenting and/or embossing the base layer 7 and/or cover layer 8.

It has to be noted that the storage members 6 could also be arranged on the carrier 5, if desired even without any (common) base layer 7 or cover layer 8.

In order to open the storage member 6, the base layer 7 and the cover layer 8 are preferably moved or guided or torn away from each other, in particular in opposite directions e.g. as shown in Fig. 1 and 4. However, the layers 7 and 8 could also be moved, guided and/or torn into the same direction as schematically shown in the further embodiment according to Fig. 5. Here, the duct 10 for discharging the formulation 2 may be opened by rupturing the base layer 7 or cover layer 8 and/or by bending, flexing or folding the carrier 5. In particular, this opening can also be achieved or supported by guiding the carrier 5 around a corner or roll with a very small radius such as one or two millimeters or less. The opening may also be supported or defined by a predetermined breaking point 19, preferably formed by weakening or perforating the material, in particular the layer 7 or 8, forming a chamfer indentation or notch or the like. This will result in a defined tracking or rupturing of the carrier 5, in particular of only one of the layers 7 and 8, at the desired point or portion.

Fig. 6 shows in a partial sectional view of the opened carrier 5 that the breaking point 19 can also be used when the layers 7 and 8 are not moved in the same direction for opening the carrier 5 or the respective storage member 6 / duct 10.

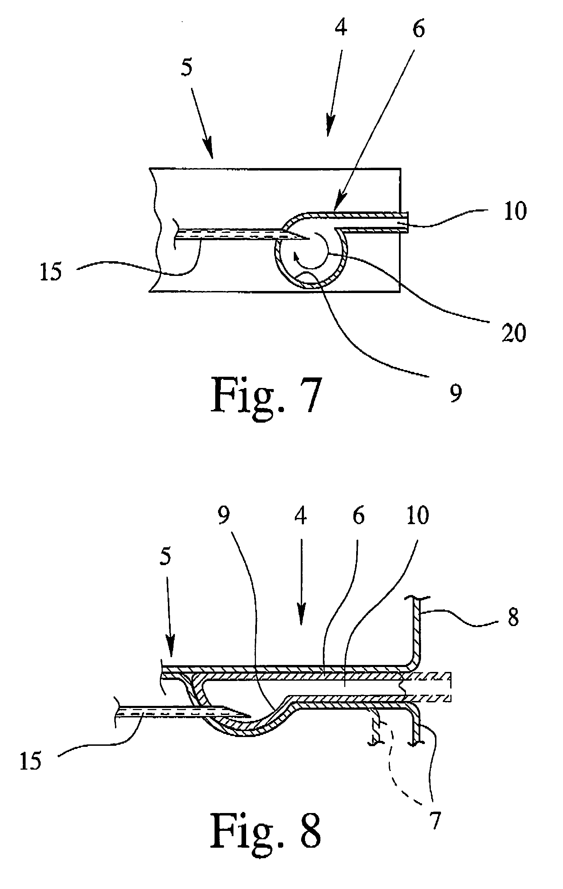

Fig. 7 and 8 show schematic sectional views of a part of the carrier 5 with pierced storage member 6 according to a preferred embodiment. Fig. 7 is a view from above or below. Fig. 8 is a sectional view perpendicular to Fig. 7.

The piercing element 15 ends preferably radially offset in the storage chamber 9 as shown in Fig. 7, and/or axially offset in the storage chamber 9 relative to the duct 10 as shown in Fig. 8. Thus, a swirl 20 can be generated in the storage chamber 9 as schematically shown in Fig. 7 which entranced the formulation 2 (not shown) contained in the storage chamber 9 when gas/air is guided into the storage chamber 9 via the piercing element 15.

However, other constructional solutions are possible to achieve a suitable supply of gas/air into the storage member 6, in particular its storage chamber 9.

It has to be noted that the storage member 6 or its duct 10 may end essentially at or before the pending of the carrier 5 or its layers 7, 8 or may extend beyond this point as schematically shown in Fig. 8, in particular by the dashed lines.

Further, it has to be noted that the layers 7 and 8 may also be bent - in Fig. 8 in opposite directions - at different points, i.e. offset in lengthwise direction of the carrier 5 as indicated by the dashed part of the base layer 7. Alternatively or additionally, the layers 7 and 8 may also be bent or folded with different diameters.

In general, each storage member 6 can comprise an individual cover to facilitate filling and/or individual sealing of the respective storage member 6.

### List of reference numbers:

1 dispensing device
2 formulation
3 spray
4 storage device
5 carrier
6 storage member
7 base layer
8 cover layer
9 storage chamber
10 duct
11 nozzle
12 dispensing direction
13 air pump
14 connecting mechanism
15 piercing element
16 mouthpiece
17 guide roller
18 spooling device
19 breaking point
20 swirl

## Claims

1. Dispensing device (1) for dispensing a preferably medical formulation (2), in particular containing or consisting of a drug, as a spray (3), wherein the dispensing device (1) is adapted to receive or comprises a storage device (4), the storage device (4) comprising a carrier (5) with a forming multiple storage members (6), each storage member (6) comprising a storage cavity (9) containing a dose of the formulation (2), and a duct (10) or nozzle (11) for dispensing the respective dose in a dispensing direction (12),
**characterized in**
**that** the dispensing direction (12) of the ducts (10) or nozzles (11) is directed at least essentially in a lengthwise or indexing direction of the storage device (4) or carrier (5), and/or
**that** the storage device (4) or carrier (5) is preferably a peelable blister strip, wherein the ducts (10) or nozzles (11) can be opened one after the other by peeling, pushing, cracking, cutting, folding or bending the blister strip, and/or
**that** the carrier (5) carries the storage members (6) formed separately from the carrier (5) rigidly or in a unmovable manner and is adapted that the storage members (6), the ducts (10) or the nozzles (11) can be opened without movement of the storage members (6) or a base layer (7) thereof by opening or peeling or pushing or cracking or cutting or bending or folding a preferably common sealing or cover layer (8).

2. Dispensing device according to claim 1, **characterized in that** the formulation (2) is a powder.

3. Dispensing device according to any one of the preceding claims, **characterized in that** the carrier (5) is flexible and/or band-like and/or is a blister, preferably a blister strip.

4. Dispensing device according to any one of the preceding claims, **characterized in that** the carrier (5) comprises the base layer (7) and the preferably peelable cover layer (8), wherein the storage members (6) are inserted or held between these layers (7, 8) before and/or during dispensing.

5. Dispensing device according to any one of the preceding claims, **characterized in that** the storage members (6) are parts made separately from the carrier (5).

6. Dispensing device according to one claims 1 to 3, **characterized in that** the storage members (6) are formed by the carrier (5), in particular the base layer (7) and the cover layer (8) of the carrier (5).

7. Dispensing device according to claim 6, **characterized in that** the storage member (6) are formed by embossing the base layer (7) and/or cover layer (8).

8. Dispensing device according to any one of the preceding claims, **characterized in that** each storage member (6) is a molded element, a cartridge, a capsule or a container.

9. Dispensing device according to any one of the preceding claims, **characterized in that** each storage member (6) is rigid or flexible.

10. Dispensing device according to any one of the preceding claims, **characterized in that** the dispensing device (1) comprises an opening, connecting and/or supply means (14), in particular a piercing element (15), such as a needle, for opening or piercing the storage members (6) individually and/or supplying pressurized gas to the respective storage member (6) to dispense the respective dose.

11. Dispensing device according to any one of the preceding claims, **characterized in that** the dispensing device (1) comprises means for providing pressurized gas, in particular air, for dispensing the formulation (2), preferably wherein the dispensing device (1) comprises a preferably manually operated air pump (13) as means for providing pressurized gas.

12. Dispensing device according to any one of the preceding claims, **characterized in that** the dispensing device (1) is designed such that the carrier (5) can be moved or indexed stepwise from one storage member (6) to the next storage member (6).

13. Dispensing device according to any one of the preceding claims, **characterized in that** the dispensing device (1) is a preferably oral inhaler, in particular a dry powder inhaler.

14. Dispensing device according to any one of the preceding claims, **characterized in that** the storage members (6), ducts (10) or nozzles (11) are sealed by one common sealing or cover layer (8).

15. Storage device (4) for a preferably medical formulation (2), in particular containing or consisting of a drug, preferably for a dispensing device (1) according to any one of the preceding claims, the storage device (4) comprising a carrier (5) with multiple storage members (6), each storage member (6) comprising a storage cavity (9) containing a dose of the formulation (2), and a duct (10) or nozzle (11) for dispensing the respective dose in a dispensing direction (12),
**characterized in**
**that** the dispensing direction (12) of the ducts (10) or nozzles (11) is directed in a lengthwise direction of the storage device (4) or carrier (5), and/or
**that** the storage device (4) or carrier (5) is preferably a peelable blister strip, wherein the ducts (10) or nozzles (11) can be opened one after the other by peeling or pushing or cracking or cutting or bending or folding the blister strip, and/or
**that** the carrier (5) carries the storage members (6) formed separately from the carrier (5) rigidly or in a unmovable manner and is adapted that the storage members (6), the ducts (10) or the nozzles (11) can be opened without movement of the storage members (6) or a base layer (7) thereof by opening or peeling a preferably common feeling or cover layer (8).

16. Storage device according to claim 15, **characterized in that** the formulation (2) is a liquid, in particular a solution, suspension or suslution.

17. Storage device according to claim 15, **characterized in that** the formulation (2) is a powder.

18. Storage device according to any one of claims 15 to 17, **characterized in that** the carrier (5) is flexible and/or band-like.

19. Storage device according to any one of claims 15 to 18, **characterized in that** the carrier (5) comprises a base layer (7) and preferably a peelable cover layer (8), wherein the storage members (6) are inserted or held between these layers (7, 8) before and/or during dispensing.

20. Storage device according to any one of claims 15 to 19, **characterized in that** the storage members (6) are parts made separately from the carrier (5).

21. Storage device according to any one of claims 15 to 18, **characterized in that** the storage members (6) are formed by the carrier (5), in particular a base layer (7) and a cover layer (8) of the carrier (5).

22. Storage device according to any one of claims 15 to 21, **characterized in that** each storage member (6) is a molded element, a cartridge, a capsule or a container.

23. Storage device according to any one of claims 15 to 22, **characterized in that** each storage member (6) is rigid or flexible.

24. Storage device according to any one of claims 15 to 23, **characterized in that** the storage members (6), ducts (10) or nozzles (11) are sealed by one common sealing or cover layer (8).

25. Method for dispensing a preferably medical formulation (2), in particular containing or consisting of a drug, as a spray (3) from a blister strip comprising with multiple storage members (6), each storage member (6) comprising a storage cavity (9) containing a dose of the formulation (2), and a duct (10) or nozzle (11) for dispensing the respective dose,
**characterized in**
**that** the blister strip is peeled pushed, cracked, bent or folded to open the storage members (6), the ducts (10) or the nozzles (11) one after the other so that the doses can be dispensed one after the other.

26. Method according to claim 25, **characterized in that** the storage members (6), ducts (10) or nozzles (11) are sealed by one common sealing or cover layer (8), wherein the common sealing or cover layer (8) is peeled, pushed, cut or cracked.

27. Method according to claim 25 or 26, **characterized in that** the storage members (6) are individually connected to a supply of pressurized gas, in particular by piercing the respective storage member (6) by means of a piercing element (15).

28. Method according to any one of claims 25 to 27, **characterized in that** the doses are individually subjected to a gas pressure for dispensing.

29. Method according to claim 28, **characterized in that** air is pressurized and used as gas, in particular wherein air is pressurized by manually operating an air pump (13).

30. Method according to any one of claims 25 to 29, **characterized in that** the spray (3) is generated directly or only by the ducts (10) and/or nozzles (11).
